Europäisches Patentamt

European Patent Office

Office européen des brevets

(11) Publication number: **0 602 691 A2**

# EUROPEAN PATENT APPLICATION

(21) Application number: **93202796.4**

(22) Date of filing: **30.09.93**

(51) Int. Cl.⁵: **A61K 49/04**

(30) Priority: **15.12.92 US 990873**

(43) Date of publication of application:
**22.06.94 Bulletin 94/25**

(84) Designated Contracting States:
**AT BE CH DE DK ES FR GB GR IE IT LI LU MC NL PT SE**

(71) Applicant: **STERLING WINTHROP INC.**
**90 Park Avenue**
**New York, NY 10016(US)**

(72) Inventor: **Illig, Carl R., c/o STERLING WINTHROP INC.**
**Patent Department,**
**90 Park Avenue**
**New York, New York 10016(US)**
Inventor: **Liversidge, Gary G., c/o STERLING WINTHROP INC.**
**Patent Department,**
**90 Park Avenue**
**New York, New York 10016(US)**
Inventor: **Mcintire, Gregory L., c/o STERLING WINTHROP INC.**
**Patent Department,**
**90 Park Avenue**
**New York, New York 10016(US)**
Inventor: **Van Orman, Barbara B., c/o STERLING WINTHROP INC.**
**Patent Department,**
**90 Park Avenue**
**New York, New York 10016(US)**

(74) Representative: **Haile, Helen Cynthia et al**
**Kodak Limited**
**Patent Department**
**Headstone Drive**
**Harrow, Middlesex HA1 4TY (GB)**

(54) Controlled precipitation of particulate diagnostic imaging contrast agents.

(57) A composition comprising a co-precipitate of a relatively solvent-soluble organic material and a relatively solvent-insoluble organic material, said relatively solvent-soluble organic material being selectively dissolved to produce a void-containing particle is described. The voids are preferably filled with air and the preferred relatively solvent-soluble organic material is carboxylic acid such as benzoic acid, salicylic acid, or diatrizoic acid and the preferred relatively solvent-insoluble organic material is an ester of diatrizoic acid, such as the ethyl ester thereof.

A method of making a void-containing particle and a method of diagnosis comprising the administration to a mammal of a contrast-effective amount of a void-containing or air-containing particle is also described.

FIELD OF THE INVENTION

The present invention is directed to a co-precipitate of two organic materials, wherein one of those organic materials is selectively solubilized.

BACKGROUND OF THE INVENTION

Ultrasonic imaging depends upon a volume of trapped air or other gas to provide sufficient contrast with surrounding tissues in order to form an ultrasound image. In conventional ultrasonic imaging, agents which generate microbubbles are used to enhance the ultrasonic agent (see e.g. U.S. Patent No. 4,681,119).

Similarly, x-ray imaging depends upon the use of a radiopaque agent. In x-ray imaging, transmitted radiation is used to produce a radiograph based upon overall tissue attenuation characteristics. X-rays pass through various tissues and are attenuated by scattering, i.e., reflection or refraction or energy absorption. However, certain body organs, vessels and anatomical sites exhibit so little absorption of x-ray radiation that radiographs of these body portions are difficult to obtain. To overcome this problem, radiologists routinely introduce an x-ray absorbing medium containing a contrast agent into such body organs, vessels and anatomical sites.

Magnetic resonance (MR) imaging depends upon the spin-lattice ($T_1$) and/or the spin-spin ($T_2$) relaxation time of protons in different tissues. Contrast agents for MR imaging include paramagnetic materials such as chelates of gadolinium and materials possessing magnetic moments.

The present invention provides a novel particulate formulation which provides high contrast useful in x-ray and MR imaging, as well as providing trapped air or other gas useful in ultrasound imaging.

BRIEF DESCRIPTION OF THE PRESENT INVENTION

The present invention is directed to a composition composed of two co-precipitated organic materials, one of which is selectively dissolved at a later time to produce a void-containing particle. In a preferred embodiment, the void-containing particle is freeze-dried to fill the voids with air to produce an air-filled particle.

The present invention is also directed to a method of making a void-containing particle, comprising co-precipitating two organic materials under conditions and for a time sufficient to form a co-precipitate, and contacting said co-precipitate with a solvent for a time and under conditions sufficient to dissolve one of the organic materials to form a void-containing particle. In a preferred embodiment, the void-containing particle is further subjected to freeze-drying for a time and under conditions sufficient to form an air-filled particle. In another preferred embodiment, the void-containing particle is reduced in size, e.g. by wet grinding.

The present invention is further directed to a method of diagnosis comprising administering to a mammal a contrast-effective amount of a void-containing particle, and generating a diagnostic image of said mammal. In a preferred embodiment, the diagnostic image is an x-ray image. In another embodiment the diagnostic image is an MR image.

The present invention is still further directed to a method of diagnosis comprising administering to a mammal a contrast-effective amount of an air-filled particle, and generating a diagnostic image of said mammal. In a preferred embodiment, the diagnostic image is an ultrasound image.

DETAILED DESCRIPTION OF THE INVENTION

The void-containing particles of this invention are hereinafter described in connection with their preferred utility, i.e. as diagnostic agents. In addition, the particles of this invention are also useful in other applications, e.g. as therapeutic and cosmetic agents.

According to the present invention there is provided a composition comprising a co-precipitate of a relatively solvent-soluble organic material and a relatively solvent-insoluble organic material, said relatively solvent-soluble organic material being selectively dissolved to produce a void-containing particle.

A co-precipitate is formed when one organic material is introduced into a solution of a second organic material such that a precipitate is formed which comprises both organic materials. As is well known in the art, the formation of a co-precipitate is dependent primarily upon the concentration of the organic materials used to form the co-precipitate, as well as other factors such as temperature, pH, time, and the like.

As used herein, a "relatively solvent-soluble organic material" is an organic material that is soluble in a particular solvent system, relative to an organic material which is insoluble in such solvent system. In a preferred embodiment, the relatively solvent-soluble organic material is a carboxylic acid, such as benzoic acid, salicylic acid or diatrizoic acid.

Analogously, as used herein, a "relatively solvent-insoluble organic material" is an organic material which is insoluble in the same solvent system, relative to an organic material which is soluble in such a solvent system. In a preferred embodiment, the relatively solvent-insoluble organic material is any poorly soluble x-ray contrast agent such as an ester, e.g., the ethyl ester of diatrizoic acid.

Solubility, as is well known in the art, is dependent upon such factors as pH, temperature, the solvent used, and the like. The solubilities of the organic materials useful in the present invention are therefore dependent on these factors, with a primary emphasis on the particular solvent used.

By the judicious choice of solvent, one of the organic materials useful herein (the relatively solvent-soluble organic material) can be selectively solubilized, leaving a particle comprised primarily of the relatively solvent-insoluble material, and voids in the spaces where the relatively solvent-soluble material existed in the co-precipitate prior to solubilization.

These voids may be filled with air or any other gas. In a preferred embodiment, the voids are filled with air. This can most easily be accomplished by freeze-drying the void-containing particles (i.e. lyophilization) for a time and under conditions sufficient to fill said voids with air or gas, preferably air. Thus the freeze-drying essentially desiccates the void-containing particles in a vacuum. Upon reconstitution of the freeze-dried particles, the voids are filled with air. Similarly, if the freeze-dried particles are reconstituted in the presence of another gas, for example, argon, the voids of the void-containing particles will be filled with argon gas.

For ultrasound imaging the mean particle size can be from 1 to about 20 $\mu$m, preferably from 1 to about 12 $\mu$m.

In a preferred embodiment for x-ray and MR imaging, the particles of the present invention have an average particle size of less than about 400 nanometers. As used herein, particle size refers to a weight average particle size as measured by conventional particle size measuring techniques well known to those skilled in the art, such as sedimentation field flow fractionation, photon correlation spectroscopy, or disk centrifugation. By "an average particle size of less than about 400 nm" it is meant that at least 90% of the particles have a weight average particle size of less than about 400 nm when measured by the above-noted techniques. In preferred embodiments of the invention, the effective average particle size is less than about 300 nm, and more preferably less than about 250 nm. In some embodiments of the invention, an effective average particle size of less than about 200 nm has been achieved. With reference to the effective average particle size, it is preferred that at least 95% and, more preferably, at least 99% of the particles have a particle size less than the effective average, e.g. 400 nm. In particularly preferred embodiments, essentially all of the particles have a size less than 400 nm. In some embodiments, essentially all of the particles have a size less than 250 nm.

The present invention includes the void-containing or air-containing particles, as hereindescribed, formulated into compositions together with one or more non-toxic physiologically acceptable carriers, adjuvants or vehicles which are collectively referred to herein as carriers, for parenteral injection, for oral administration in solid or liquid form, for rectal or topical administration, for inhalation or the like.

The compositions can be administered to humans and animals either orally, rectally, parenterally (intravenous, intramuscular or subcutaneous), intracisternally, intravaginally, intraperitoneally, locally (powders, ointments or drops), or as a buccal or nasal spray.

Compositions suitable for parenteral injection may comprise physiologically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethylene glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride and the like.

Solid dosage forms for oral administration include capsules, tablets, pills, powders and granules. In such solid dosage forms, the active compound is admixed with at least one inert customary excipient (or carrier) such as sodium citrate or dicalcium phosphate or (a) fillers or extenders, e.g. starches, lactose, sucrose, glucose, mannitol and silicic acid, (b) binders, e.g. carboxymethylcellulose, alignates, gelatin, polyvinylpyrrolidone, sucrose and acacia, (c) humectants, e.g. glycerol,(d) disintegrating agents, e.g. agar-agar, calcium carbonate, potato or tapioca starch, alginic acid, certain complex silicates and sodium carbonate, (e) solution retarders, e.g. paraffin, (f) absorption accelerators, e.g. quaternary ammonium compounds, (g) wetting agents, e.g. cetyl alcohol and glycerol monostearate, (h) adsorbents, e.g. kaolin and bentonite, and (i) lubricants, e.g. talc, calcium stearate, magnesium stearate, solid polyethylene glycols, sodium lauryl sulfate or mixtures thereof. In the case of capsules, tablets and pills, the dosage forms may also comprise buffering agents.

Solid compositions of a similar type may also be employed as fillers in soft and hard-filled gelatin capsules using such excipients as lactose or milk sugar as well as high molecular weight polyethyleneglycols, and the like.

Solid dosage forms such as tablets, dragees, capsules, pills and granules can be prepared with coatings and shells, such as enteric coatings and others well known in the art. They may contain opacifying agents, and can also be of such composition that they release the active compound or compounds in a certain part of the intestinal tract in a delayed manner. Examples of embedding compositions which can be used are polymeric substances and waxes.

The active compounds can also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, N,N-dimethylformamide, oils, in particular, cottonseed oil, groundnut oil, corn germ oil, olive oil, castor oil and sesame oil, glycerol, tetrahydrofurfuryl alcohol, polyethylene glycols and fatty acid esters of sorbitan or mixtures of these substances, and the like.

Besides such inert diluents, the composition can also include adjuvants, such as wetting agents, emulsifying and suspending agents, sweetening, flavoring and perfuming agents.

Suspensions, in addition to the active compounds, may contain suspending agents, e.g. ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are preferably suppositories which can be prepared by mixing the compounds of the present invention with suitable non-irritating excipients or carriers such as cocoa butter, polyethyleneglycol or a suppository wax, which are solid at ordinary temperatures but liquid at body temperature and therefore, melt in the rectum or vaginal cavity and release the active component.

Dosage forms for topical administration of a compound of this invention include ointments, powders, sprays and inhalants. The active component is admixed under sterile conditions with a physiologically acceptable carrier and any preservatives, buffers or propellants as may be required. Ophthalmic formulations, eye ointments, powders and solutions are also contemplated as being within the scope of this invention.

Actual dosage levels of active ingredients in the compositions of the present invention may be varied so as to obtain an amount of active ingredient that is effective to obtain a desired therapeutic response for a particular composition and method of administration. The selected dosage level therefore depends upon the desired therapeutic effect, on the route of administration, on the desired duration of treatment and other factors.

The total daily dose of the compounds of this invention administered to a host in single or divided dose may be in amounts, for example, of from about 1 nanomole to about 5 micromoles per kg body weight. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

According to a further aspect of the present invention there is provided a method of making a void-containing particle, comprising co-precipitating a relatively solvent-soluble organic material and a relatively solvent-insoluble organic material under conditions and for a time sufficient to form a co-precipitate, and contacting said co-precipitate with a solvent for a time and under conditions sufficient to dissolve said relatively solvent-soluble organic material to form a void-containing particle.

The amount of relatively solvent-soluble material used to form the co-precipitate depends upon a variety of conditions, such as temperature, solubility, molar concentration of the relatively solvent-insoluble material, and the like. A preferred amount of relatively solvent-soluble material is 5% to about 90%, more preferably 10% to about 80%.

The amount of relatively solvent-insoluble material used to form the co-precipitate similarly depends upon such conditions, including however in this instance the molar concentration of the relatively solvent-soluble material. A preferred amount of relatively solvent-insoluble material is 10% to about 95%, more preferably 20% to about 90%.

The co-precipitate comprising the relatively solvent-soluble material and the relatively solvent-insoluble material is therafter contacted with the solvent appropriate for the solubilities of the organic materials. That is, the solvent which is contacted with the co-precipitate is one which will solubilize the relatively solvent-soluble organic material, while not solubilizing the relatively solvent-insoluble organic material. In a preferred embodiment, an aqueous solvent with a pH greater than seven is employed to solubilize a carboxylic acid, such as benzoic acid, used as the solvent soluble component.

Once the co-precipitate is contacted with the solvent of choice, the relatively solvent-soluble organic material is solubilized from the milieu of the surrounding relatively solvent-insoluble organic material, thereby generating voids in the relatively solvent-insoluble organic material.

It is a particular advantage of this invention that, in some instances, the void containing particles obtained after solubilization of the solvent soluble material are structurally fragile. As a result, these particles can be further reduced in size by various comminution techniques, preferably by wet-grinding as described below.

In one embodiment, the particles of the present invention, whether void-containing particles or air-containing particles, have an average particle size of less than about 400 nm, as discussed elsewhere herein. The particles, sometimes referred to herein as nanoparticles, useful in the practice of this invention, can be prepared according to the methods disclosed in U.S. Patent No. 5,145,684. Briefly, nanoparticles are prepared by dispersing a poorly soluble therapeutic or diagnostic agent in a liquid dispersion medium and wet-grinding the agent in the presence of grinding media to reduce the particle size of the contrast agent to an effective average particle size of less than about 400 nm. The particles can be reduced in size in the presence of a surface modifier.

A general procedure for preparing the particles useful in the practice of this invention follows. The therapeutic or diagnostic agent selected is obtained commercially and/or prepared by techniques known in the art as described above, in a conventional coarse form. It is preferred, but not essential, that the particle size of the coarse therapeutic or diagnostic substance selected be less than about 100 $\mu$m as determined by sieve analysis. If the coarse particle size of that agent is greater than about 100 $\mu$m, then it is preferred that the coarse particles of the therapeutic or diagnostic agent be reduced in size to less than 100 $\mu$m using a conventional milling method such as airjet or fragmentation milling.

The coarse therapeutic or diagnostic agent selected can then be added to a liquid medium in which it is essentially insoluble to form a premix. The concentration of the therapeutic or diagnostic agent in the liquid medium can vary from about 0.1-60%, and preferably is from 5-30% (w/w). It is preferred, but not essential, that the surface modifier be present in the premix. The concentration of the surface modifier can vary from about 0.1 to 90%, and preferably is 1-75%, more preferably 10-60% and most preferably 10-30% by weight based on the total combined weight of the drug substance and surface modifier. The apparent viscosity of the premix suspension is preferably less than about 1000 centipoise.

The premix can be used directly by wet grinding to reduce the average particle size in the dispersion to less than 400 nm. It is preferred that the premix be used directly when a ball mill is used for attrition. Alternatively, the therapeutic or diagnostic agent and, optionally, the surface modifier, can be dispersed in the liquid medium using suitable agitation, e.g. a roller mill or a Cowles type mixer, until a homogeneous dispersion is observed in which there are no large agglomerates visible to the naked eye. It is preferred that the premix be subjected to such a premilling dispersion step when a recirculating media mill is used for attrition.

Wet grinding can take place in any suitable dispersion mill, including, for example, a ball mill, an attritor mill, a vibratory mill, and media mills such as a sand mill and a bead mill. A media mill is preferred due to the relatively shorter milling time required to provide the intended result, i.e., the desired reduction in particle size. For media milling, the apparent viscosity of the premix preferably is from about 100 to about 1000 centipoise. For ball milling, the apparent viscosity of the premix preferably is from about 1 up to about 100 centipoise. Such ranges tend to afford an optimal balance between efficient particle fragmentation and media erosion.

The grinding media for the particle size reduction step can be selected from rigid media preferably spherical or particulate in form having an average size less than about 3 mm and, more preferably, less than about 1 mm. Such media desirably can provide the particles of the invention with shorter processing times and impart less wear to the milling equipment. The selection of material for the grinding media is not believed to be critical. However, preferred media have a density greater than about 3 g/cm$^3$. Zirconium

oxide, such as 95% ZrO stabilized with magnesia, zirconium silicate, and glass grinding media provide particles having levels of contamination which are believed to be acceptable for the preparation of therapeutic or diagnostic compositions. However, other media, such as stainless steel, titania, alumina, and 95% ZrO stabilized with yttrium, are believed to be useful.

The attrition time can vary widely and depends primarily upon the particular wet grinding mill selected. For ball mills, processing times of up to five days or longer may be required. On the other hand, processing times of less than 1 day (residence times of about one minute up to several hours) have provided the desired results using a high shear media mill. It is a particular advantage that nanoparticles can be attained with shorter milling times using the void-containing particles of this invention.

The particles must be reduced in size at a temperature which does not significantly degrade the therapeutic or diagnostic agent. Processing temperatures of less than about 30-40°C are ordinarily preferred. If desired, the processing equipment can be cooled with conventional cooling equipment. The method is conveniently carried out under conditions of ambient temperature and at processing pressures which are safe and effective for the milling process. For example, ambient processing pressures are typical of ball mills, attritor mills and vibratory mills. Processing pressures up to about 1.4 $kg/cm^2$ are typical of media milling.

The surface modifier, if not present in the premix, must be added to the dispersion after attrition in an amount as described for the premix. Thereafter, the dispersion can be mixed, e.g. by shaking vigorously. Optionally, the dispersion can be subjected to a sonication step, e.g. using an ultrasonic power supply. For example, the dispersion can be subjected to ultrasonic energy having a frequency of 20-80 kHz for a time of about 1 to 120 seconds.

The relative amount of therapeutic or diagnostic agent and surface modifier can vary widely and the optimal amount of the surface modifier can depend, for example, upon the particular therapeutic or diagnostic agent and surface modifier selected, the critical micelle concentration of the surface modifier if it forms micelles, the hydrophilic lipophilic balance (HLB) of the stabilizer, the melting point of the stabilizer, its water solubility, the surface tension of water solutions of the stabilizer, etc. The surface modifier preferably is present in an amount of about 0.1-10 $mg/m^2$ surface area of the therapeutic or diagnostic agent.

The present invention is also directed to a method of diagnosis comprising administering to a mammal a contrast-effective amount of a void-containing or air-containing particle as described elsewhere herein, dissolved or dispersed in a physiologically tolerable carrier, and generating a diagnostic image of said mammal.

A method for diagnostic imaging for use in medical procedures in accordance with this invention comprises administering to the body of a test subject in need of a diagnostic image an effective contrast producing amount of the above-described void-containing composition. In addition to human patients, the test subject can include mammalian species such as rabbits, dogs, cats, monkeys, sheep, pigs, horses, bovine animals and the like.

Thereafter, in a preferred embodiment, at least a portion of the body containing the administered contrast agent is exposed to x-rays, ultrasound energy or a magnetic field to produce an x-ray or magnetic resonance image pattern corresponding to the presence of the contrast agent. The image pattern can then be visualized.

In x-ray imaging, transmitted radiation is used to produce a radiograph based upon overall tissue attenuation characteristics. Any x-ray visualization technique, preferably, a high contrast technique such as computed tomography, can be applied in a conventional manner. Alternatively, the image pattern can be observed directly on an x-ray sensitive phosphor screen-silver halide photographic film combination. Visualization with a magnetic resonance imaging system can be accomplished with commercially available magnetic imaging systems such as a General Electric 1.5 T Sigma imaging system [1H resonant frequency 63.9 MHz]. Commercially available magnetic resonance imaging systems are typically characterized by the magnetic field strength used, with a field strength of 2.0 Tesla as the current maximum and 0.2 Tesla as the current minimum.

For a given field strength, each detected nucleus has a characteristic frequency. For example, at a field strength of 1.0 Tesla, the resonance frequency for hydrogen is 42.57 MHz; for phosphorus-31 it is 17.24 MHz; and for sodium-23 it is 11.26 MHz.

In a further preferred embodiment, the test subject is exposed to ultrasonic energy for ultrasonic visualization. Any ultrasound visualization technique can be applied in a conventional manner.

A contrast effective amount of a composition of the present invention is that amount necessary to provide tissue visualization with, for example, magnetic resonance imaging, x-ray imaging, or ultrasonic imaging. Means for determining a contrast effective amount in a particular subject will depend, as is well known in the art, on the nature of the magnetically reactive material used, the mass of the subject being imaged, the sensitivity of the magnetic resonance or x-ray imaging system and the like.

After administration of a composition of the present invention, the subject mammal is maintained for a time period sufficient for the administered composition to be distributed throughout the subject and enter the tissues of the mammal. Typically, a sufficient time period is from about 20 minutes to about 90 minutes and, preferably from about 20 minutes to about 60 minutes.

The invention will now be illustrated with reference to the following examples but is in no way to be construed as limited thereto.

Example 1. Co-precipitation of ethyl 3,5-diacetamido-2,4,6-triiodobenzoate/Diatrizoic Acid

4.13 g ethyl 3,5-diacetamido-2,4,6-triiodobenzoate (6.43 mmol) and 5.92 g diatrizoic acid (9.64 mmol, 1.5 eq.) in 20.1 ml dimethylformamide (DMF) (2:1 ml DMF/g compound) was poured at 20°C into 101 ml brine containing 0.5 ml 6M HCl with vigorous stirring. The mixture was cooled to 10°C, filtered and the precipitate dried (70°C/0.1 torr) over $P_2O_5$ for 18 hr to afford 8.31 g white solid.

Void-containing particles were prepared by subjecting the co-precipitate to the procedure described in Example 2.

Example 2. Co-precipitation of ethyl 3,5-diacetamido-2,4,6-triiodobenzoate/Diatrizoic Acid

A series of experiments were run using the above ethyl ester of diatrizoic acid and diatrizoic acid in solution in DMF as the reactant being pumped into the aqueous phase. Temperature, flow rate, and salt additives to the aqueous host solution were the variables tested.

The product of the precipitation from Example 1 went through the procedure listed below before undergoing scanning electron microscopy (SEM) inspection. This procedure was:

1. Spin at 3500 rpm for 30 min.
2. Decant supernatant.
3. Rinse and resuspend with approximately 30 ml deionized water.
4. Repeat steps 1 & 2.
5. Resuspend in approximately 30 ml deionized water and adjust pH to about 10 with 0.1N NaOH.
6. Shake overnight.
7. Repeat steps 1 & 2 twice.
8. Resuspend in 10 ml of appropriate surfactant for a final 10% suspension.
9. Mill an aliquot with 1.1mm Zirconium silicate beads for 24 hr.
10. Send unmilled and milled samples for SEM analysis.

The results of these analyses are shown in Table 1.

## Table 1

| EXPERIMENTAL | RESULT |
|---|---|
| 1ml/min; room temperature (RT) | Milled to 250 nm (3% Tyloxapol) SEM: blocks with an almost smooth surface |
| 5ml/min; RT | Milled to 290 nm (3% Tyloxapol) SEM: dimpled surface polygonal |
| 1ml/min; cold | Milled to 240 nm (3% Tyloxapol) SEM: plates with holes |
| 5ml/min; cold | Milled to 290 nm (3% Tyloxapol) SEM: creviced, aggregated polygonal |
| 1ml/min; 0.5M TBAP* | Milled to 360 nm (3% Tyloxapol) SEM: plates polygonal |
| 1ml/min; 0.5M NaHPO$_4$ | Milled to 310 nm (3% Tyloxapol) SEM: jigsaw pieces. Milled to smaller, monodisperse than seen by photon correlation spectroscopy (PCS). |
| 0.5ml/min; 0.5M NaHPO$_4$ | Milled to 400 nm (3% Tyloxapol) SEM: plates, polygonal, smooth |

0.15ml/min;          Milled to 440 nm
0.5M NaHPO$_4$          (3% Tyloxapol)
                     SEM: plates, polygonal, smooth

0.5ml/min;           Milled to 400 nm
0.5M TBAP            (3% Tyloxapol/0.5% AOT**)
                     SEM: thin etched plates, polygonal

1 ml/min;            Milled to 400 nm
0.5M TBAP            (3% Tyloxapol/0.5% AOT)
                     SEM: creviced plates, polygonal

2 ml/min;            Milled to 400 nm
0.5M TBAP          (3% Tyloxapol/0.5% AOT)
                     SEM: thin plates, smooth

1 ml/min; 0.5M       Milled to 440 nm
NaHPO$_4$               (3% Tyloxapol)
                     SEM: samples taken after every
                     step.  Final are thin, creviced
                     plates, polygonal

1.0ml/min;0.5M       Milled to 450 nm
NAHPO$_4$***            (3% Tyloxapol)
                     SEM: samples taken after every
                     step.  Final are thin plates,
                     polygonal, slightly etched.

*TBAP = tetrabutylammoniumperchlorate
**AOT = Aerosol OT™, a dioctyl ester of sodium
sulfosuccinic acid, available from American Cyanamid
***Steps 5-7, above, were not done in this assay to
avoid dissolving the diatrizoic acid.

The results showed that all the samples were greater than 1 $\mu$m after the initial precipitation. Milling for 24 hrs decreased the size to as low as 240 nm. Longer milling would decrease the particle size to even smaller values.

Example 3 Co-precipitation of ethyl 3,5-diacetamido-2,4,6-triiodobenzoate/Salicylic Acid

In a manner similar to Example 1, various co-precipitates were prepared using 2:1, 1:1 and 1:2 by weight ratios of ethyl 3,5-diacetamido-2,4,6-triiodobenzoate/salicylic acid. The ratio of ml DMF to g compound mixture was 2:1. The ratio of ml water to ml DMF was 7:1.

Example 4 Co-precipitation of ethyl 3,5-diacetamido-2,4,6-triiodobenzoate/Benzoic Acid

In a manner similar to Example 1, two co-precipitates were prepared using 1:1 and 1:2 by weight ratios of ethyl 3,5-diacetamido-2,4,6-triiodobenzoate/benzoic acid. Void-containing particles were prepared by subjecting the coprecipitate to the procedure described in Example 5.

Example 5 X-Ray Imaging

The 1:2 (w/w) co-precipitate of ethyl 3,5-diacetamido-2,4,6-triiodobenzoate and salicylic acid described in Example 3 was suspended in water and then treated with 0.1N NaOH until the pH of the suspension remained stable between 8 and 10. The resulting suspension was then centrifuged for 5 min. at 3500 rpm to sediment the remaining solid particles. The supernatant was then discarded and the process repeated using fresh water. After the second centrifugation, the solid particles were resuspended using an aqueous solution of a stabilizer, in this case Tyloxapol. The suspension was then placed into a glass jar with 1 mm $ZrO_2$ media and roller milled for 24 hours.

The resulting nanoparticle suspension had a size of approximately 170 nm. Imaging studies were conducted at the Center for Imaging and Pharmaceutic Research (CIPR) at the Massachusetts General Hospital, Boston, MA. After subcutaneous injection of 0.5 ml in the forepaws of rabbits, the suspension migrated through the lymphatics vessels opacifying the regional draining lymph nodes associated with the site of injection as detected by computed tomography (CT) imaging. The intensity of the imaging was sufficient to aid in diagnostic assessment of disease.

**Claims**

1. A composition comprising a co-precipitate of a relatively solvent-soluble organic material and a relatively solvent-insoluble organic material, said relatively solvent-soluble organic material being selectively dissolved to produce a void-containing particle.

2. A composition as claimed in claim 1 wherein the voids of said void-containing particle are filled with air.

3. A composition as claimed in either of the preceding claims wherein said relatively solvent-soluble organic material is a carboxylic acid.

4. A composition as claimed in claim 3 wherein said carboxylic acid is selected from the class consisting of benzoic acid, diatrizoic acid and salicylic acid

5. A composition as claimed in either of claims 1 and 2 wherein said relatively solvent-insoluble organic material is an ester of diatrizoic acid.

6. A composition as claimed in claim 5 wherein said ester is the ethyl ester.

7. A composition as claimed in any one of the preceding claims wherein said particles have an average particle size of less than about 400 nanometers.

8. A method of making a void-containing particle, comprising co-precipitating a relatively solvent-soluble organic material and a relatively solvent-insoluble organic material under conditions and for a time sufficient to form a co-precipitate, and contacting said co-precipitate with a solvent for a time and under conditions sufficient to dissolve said relatively solvent-soluble organic material to form a void-containing particle.

9. A method as claimed in claim 8 further comprising freeze-drying said void-containing particle for a time and under conditions sufficient to fill said voids with air.

**10.** A method as claimed in claim 8 wherein said relatively solvent-soluble organic material and said relatively solvent-insoluble organic material is as claimed in any one of claims 3 to 6.

**11.** A method of diagnosis comprising administering to a mammal a contrast-effective amount of said void-containing particle as claimed in either of claims 1 and 2, dissolved or dispersed in a physiologically tolerable carrier, and generating a diagnostic image of said mammal.

**12.** A method as claimed in claim 11 wherein said diagnostic image is an x-ray image, an ultrasound image or a magnetic resonance image.